# EUROPÄISCHE PATENTSCHRIFT

(11) **EP 2 175 809 B1**
(45) Veröffentlichungstag und Bekanntmachung des Hinweises auf die Patenterteilung: **07.09.2016**
(21) Anmeldenummer: 08784314.0
(22) Anmeldetag: 09.07.2008
(51) Int. Cl.: A61F 2/64, A61F 2/50, A61F 2/76, A61F 2/68, A61F 2/60

(54) **PROTHESE ODER ORTHESE MIT EINEM ORTHOPÄDIETECHNISCHEN FLUIDDÄMPFER**
PROSTHESIS OR ORTHOSIS HAVING AN ORTHOPAEDIC FLUID DAMPER
PROTHÈSE OU ORTHÈSE AVEC UN AMORTISSEUR À FLUIDE ORTHOPEDIQUE

(30) Priorität: 09.07.2007 DE 102007032090
(43) Veröffentlichungstag der Anmeldung: 21.04.2010
(73) Patentinhaber: Otto Bock HealthCare GmbH, 37115 Duderstadt (DE)
(72) Erfinder: PUSCH, Martin, 37115 Duderstadt (DE); NÖRTHEMANN, Jens, 37115 Duderstadt (DE); KETTWIG, Thomas, 37085 Göttingen (DE)
(74) Vertreter: Stornebel, Kai
(86) Internationale Anmeldenummer: PCT/DE2008/001128
(87) Internationale Veröffentlichungsnummer: WO 2009/006886

(56) Entgegenhaltungen:
- EP-A- 0 309 441
- EP-A- 0 654 254
- EP-A- 1 348 409
- EP-A1- 1 736 121
- WO-A-2007/016408
- DE-A1- 3 028 608
- DE-A1- 19 859 931
- DE-A1-102005 020 188
- US-A- 5 092 902

## Beschreibung

Die Erfindung betrifft eine Prothese oder Orthese mit einem Orthopädietechnischen Dämpfermit einer in einem Zylindergehäuse ausgebildeten Verdrängungskammer, einem in der Verdrängungskammer gelagerten Kolben, einem Fluidreservoir für ein Fluid, einer Rückstromleitung, die die Verdrängungskammer mit dem Fluidreservoir verbindet, und einem Ventil, das eine Öffnungsstellung und eine Schließstellung, in der es die Rückstromleitung zumindest teilweise verschließt, einnehmen kann. Gemäß einem weiteren Aspekt betrifft die Erfindung ein Verfahren zum Steuern der Prothese oder Orthese.

Orthopädietechnische Fluiddämpfer werden in orthopädischen Hilfsmitteln, wie beispielsweise Prothesen oder Orthesen, eingesetzt. Sie dienen als Widerstandselement zwischen zwei Schenkeln. Beispielsweise ist der orthopädietechnische Fluiddämpfer zwischen einem Oberschenkel und einem Unterschenkel einer Beinprothese angeordnet und erhöht einen Beugewiderstand eines Kniegelenks der Beinprothese.

Selten findet man Beinprothesen mit derartigen orthopädietechnischen Fluiddämpfern bei geriatrischen Patienten. Für Patienten hohen Alters, die in ihrer muskulären und motorischen Leistungsfähigkeit stark eingeschränkt sind, werden häufig Sperrkniegelenke eingesetzt.

Bekannte orthopädietechnische Sperrkniegelenke für geriatrische Beinprothesen stellen den geriatrischen Patienten aus Kostengründen nur zwei Zustände, gesperrt oder entsperrt, zur Verfügung. Mit dem mittels der Sperre gesperrten Sperrkniegelenk geht der geriatrische Patient vorzugsweise. Bekannte orthopädietechnische Sperrgelenke haben den Nachteil, dass sie dazu führen, dass der Gang des Patienten humpelnd erscheint, da er das Bein außenrotiert (Zirkumduktion) nach vorn bringt oder über eine Fußspitzstellung oder über Hüftschrägstellung das gesunde Bein verlängert, um die Beinprothese frei durchschwingen zu können. Um sich hinsetzen zu können, entriegelt der geriatrische Patient die Sperre manuell. In diesem Zustand bietet die Beinprothese keinen Beugewiderstand und damit nachteiligerweise auch keine Sicherheit mehr. Der Patient muss sich in dieser Situation beidhändig abstützen. Sollte der geriatrische Patient zudem mit der entriegelten Beinprothese stolpern, stürzt er unwillkürlich. Aus diesem Grund treffen bekannte orthopädische Hilfsmittel, die mit herkömmlichen orthopädietechnischen Sperren ausgestattet sind, auf wenig Akzeptanz bei geriatrischen Patienten.

Aus der EP 0 309 441 ist eine doppelt wirkende hydraulische Kolben-Zylinder-Einheit bekannt, mit der die Außenabmessungen eines Balgs eine etwa vorgegebene Form beibehalten, wobei der Balg große Teile der Kolben-Zylinder-Einheit umspannt. Nachteilig an dieser Kolben-Zylinder-Einheit ist die aufwändige Herstellung.

Aus der DE 102 14 357 A1 ist ein Prothesen-Kniegelenk mit einem hydraulischen Dämpfungszylinder bekannt. In dem Prothesen-Kniegelenk ist eine Hydraulikflüssigkeit vorhanden, die über ein externes Kraftfeld in ihrer Viskosität veränderbar ist. Nachteilig an dieser Ausführungsform ist, dass sie zwar einen erhöhten Gehkomfort ermöglicht, dafür aber eine elektrische Steuerung benötigt, was aber für niedrigpreisige Prothesen nicht vertretbar ist.

Aus der DE 198 59 931 A1 ist eine Beinprothese bekannt, die ebenfalls über eine durch externe elektrische Felder in ihrer Viskosität veränderbare Hydraulikflüssigkeit mehr oder weniger stark gedämpft wird. Nachteilig ist auch hier, dass eine Steuerung notwendig ist.

Die DE 10 2005 020 188 A1 beschreibt ein mechatronisches System mit einem Piezoventil, einem Piezosensor und einem Verbindungsnetz, das den Sensor mit dem Ventil elektrisch verbindet. Der Sensor erzeugt ein elektrisches Signal, wenn eine Kraft auf ihn ausgeübt wird und das Ventilelement führt in Abhängigkeit von dem Signal eine Ventilbewegung aus.

Die WO 2007/016408 A1 beschreibt ein Prothesenkniegelenk mit einem hydraulischen Dämpfer, einem Mikroprozessor und Sensoren. Auf der Grundlage der Sensorwerte wählt der Mikroprozessor einen Strömungsweg innerhalb des Hydraulikzylinders, um einen geeigneten Flexionswiderstand bereitzustellen.

Ein Fluidreservoir ist über mehrere Ventile mit den Verdrängungskammern gekoppelt.

Die EP 1 736 121 A1 betrifft eine hydraulische Kniegelenkprothese mit einer Gelenkeinheit, einer Fußanschlusseinheit und einem beide Komponenten verbindenden Rohr, wobei die Gelenkeinheit eine Drehachse mit einer Dämpferkammer und einem darin bewegbaren Dämpferflügel beinhaltet, die von einer Zentraleinheit einer Hydrauliksteuerung angesteuert ist. Die Dämpferkammer ist in eine Streckkammer und eine Beugekammer unterteilt, die über eine Ölleitung miteinander verbunden sind, die durch einen Ventilkolben ganz oder teilweise verschließbar ist. Die Fußanschlusseinheit ist so ausgelegt, dass ein Auftreten mit dem Fuß bodennah als solches erkannt wird und ein hydraulisches Signal über eine Steuerleitung zum Sperren der Dämpferkammer ausgelöst wird.

Der Erfindung liegt die Aufgabe zugrunde, einen orthopädietechnischen Fluiddämpfer zu schaffen, mit dem orthopädische Hilfsmittel herstellbar sind, die bei geriatrischen Patienten auf eine höhere Akzeptanz stoßen.

Die Erfindung löst das Problem durch eine Prothese oder Orthese mit den Merkmalen des Hauptanspruchs Gemäß einem zweiten Aspekt löst die Erfindung das Problem durch ein Verfahren zum Steuern einer solchen Prothese oder Orthese mit den Merkmalen des nebengeordneten Anspruchs 10. Die Prothese oder Orthese mit einem orthopädietechnischen Fluiddämpfer mit einer in einem Gehäuse ausgebildeten Verdrängerkammer, einem in der Verdrängerkammer gelagerten Kolben, einem Fluidreservoir für ein Fluid, eine Rückstromleitung, die die Verdrängerkammer mit dem Fluidreservoir verbindet, einem Gelenk, das einen ersten Schenkel und einen zweiten Schenkel aufweist, wobei der erste Schenkel mit dem Gehäuse und der zweite Schenkel mit dem Kolben verbunden ist, einem Ventil, das eine Öffnungsstellung und eine Schließstellung, in der es die Rückstromleitung zumindest teilweise verschließt, einnehmen kann und einer Vorrichtung zum Erfassen einer auf das Gelenk wirkenden Gelenkkraft, die ausgebildet ist, um das Ventil in die Schließstellung zu bringen, wenn die Gelenkkraft einen voreingestellten Schwellenwert überschreitet, sieht vor, dass der Dämpfer und das Gelenk gemeinsam an einem Rahmen gelagert sind oder diesen bilden, der relativ zu einem der Schenkel verlagerbar, insbesondere verschiebbar ist. Das Verfahren zum Steuern eines solchen orthopädischen Hilfsmittels sieht das Erfassen einer auf das Gelenk wirkenden Gelenkkraft und das Ansteuern des Ventils so vor, dass es die Rückstromleitung zumindest teilweise verschließt. Ein orthopädietechnischer Fluiddämpfer, der zur Verwendung in einem solchen orthopädischen Hilfsmittel in Gestalt einer Prothese oder Orthese ausgebildet ist, mit einer in einem Gehäuse ausgebildeten Verdrängungskammer, einem in der Verdrängungskammer gelagerten Kolben, einem Fluidreservoir für ein Fluid, einer Rückstromleitung, die die Verdrängungskammer mit dem Fluidreservoir verbindet und einem Ventil, das eine Öffnungsstellung und eine Schließstellung, in der es die Rückstromleitung zumindest teilweise verschließt, einnehmen kann, sieht vor, dass der Kolben unter Bildung eines Ringspaltes in der Verdrängungskammer gelagert ist.

Die Prothese oder Orthese umfasst bevorzugt eine Vorrichtung zur Erfassung der auf die Prothese wirkenden Belastung, die ausgebildet ist, um das Ventil in die Schließstellung zu bringen, wenn die Gelenkkraft einen voreingestellten Schwellenwert überschreitet. Diese Vorrichtung kann beispielsweise ein Sensor sein, der eine Kraft oder ein Drehmoment erfasst. Die Vorrichtung kann jedoch auch durch eine rein mechanische Verbindung gebildet sein.

Die Prothese oder Orthese ist vorzugsweise eine Beinprothese mit einem Kniegelenk und einer Vorrichtung zur Erfassung der auf die Prothese wirkenden Belastung. Diese Vorrichtung umfasst einen Beinachsenkraftsensor, der die in Längsrichtung der gestreckten Beinprothese wirkende Beinachsenkraft erfasst.

Um die Beinachsenkraft besonders genau und feinfühlig ermitteln zu können, sind bevorzugt ein in einem Vorderfuß eines Fußes der Beinpröthese angeordneter Vorderfußkraftsensor und/oder ein in einem Hinterfuß angeordneter Hinterfußkraftsensor angeordnet. Diese stehen in elektrischer Verbindung mit einer elektrischen Steuerung, die das Ventil in Abhängigkeit von elektrischen Signalen ansteuert, die sie von dem Sensor bzw. den Sensoren erhält.

Der Beinachsenkraftsensor kann als tragendes Element der Prothese ausgebildet sein, das beispielsweise seine Längsausdehnung in Abhängigkeit von der Belastung der Beinprothese ändert. Alternativ kann der Beinkraftsensor Dehnungsmessstreifen umfassen.

Alternativ zu einer elektronischen Messung, Auswertung und Aktuierung ist es vorgesehen, dass die Vorrichtung zur Erfassung der Gelenkkraft mechanisch aufgebaut ist und das Ventil bei einer Belastung in die Schließstellung verlagert. Die mechanische Steuerung kann beispielsweise über eine Relativverlagerung der Schenkel zueinander erfolgen, indem ein Verschwenken oder eine Axialverlagerung der Schenkel zueinander ausgenutzt und die auftretenden Kräfte zur Verlagerung des Ventils auf dieses zumindest teilweise übertragen werden. Dazu kann die Vorrichtung zur Erfassung der Gelenkkraft als eine Knickfeder, Federlasche, Nockenführung, Kulissenführung oder als ein Hebelsystem ausgebildet sein, über die die Verlagerungskräfte auf das Ventil übertragen werden. Bevorzug ist die Vorrichtung mit einem Magnetventil gekoppelt, das in Richtung auf die Schließstellung vorgespannt ist, so dass bei einem Entfernen eines Schaltmagneten automatisch das Ventil geschlossen wird. Hierdurch wird sichergestellt, dass bei einem Ausfall der Mechanik die Dämpfung schlagartig erhöht wird, wodurch die Stabilität des Gelenkes gewährleistet ist.

Die Schaltbewegung kann dadurch erfolgen, dass bei einer ausreichend groβen Axialkraftkomponente die Schenkel aufeinander zu verschoben werden. Dazu sind die beiden Schenkel relativ zueinander begrenzt verschieblich aneinander gelagert, wodurch bei einer mechanischen Schaltung unmittelbar das Ventil aktuiert wir, während bei einer Sensorschaltung der Verschiebeweg als zu sensierende Größe erfasst wird, über die ein Aktuatorsignal ausgelöst wird. Die Verschiebung zeigt eine Axialkraftbelastung an, beispielsweise wenn ein Prothesennutzer steht, so dass eine Standphasensicherung über eine Dämpfungserhöhung erfolgen kann. Der Verschiebebewegung entgegenwirkend kann eine in Axialrichtung wirkende Feder vorgesehen sein, die Toleranzen ausgleicht und eine Rückstellbewegung in den Ausgangszustand bewirkt.

Die Erfindung sieht vor, dass der Dämpfer und das Gelenk gemeinsam an einem Rahmen gelagert sind oder diesen bilden, der relativ zu einem der Schenkel verlagerbar, insbesondere verschiebbar ist. Zwischen dem Rahmen und einem der Schenkel kann eine Feder angeordnet sein, die entgegen einer Verlagerung des Schenkels auf den Rahmen zu wirkt. Der Hydraulikdämpfer kann als einfach- oder doppeltwirkender, als Zylinderdämpfer oder als Rotationshydraulik ausgebildet sein.

Neben einer Axialkraft abhängigen Steuerung ist es zusätzlich möglich und vorgesehen, dass die Vorrichtung so ausgebildet ist, dass eine von dem Schwenkwinkel ϕ abhängige Verlagerung eines Schaltelementes relativ zu dem Ventil erfolgt, bevorzugt aber nicht ausschließlich mechanisch gekoppelt.

Im Rahmen eines erfindungsgemäßen Verfahrens wird das Ventil bevorzugt so angesteuert, dass es die Rückstromleitung zumindest teilweise verschließt, wenn die Beinachsenkraft einen voreingestellten Schließ-Schwellenwert übersteigt. Hierzu ist es möglich, nicht aber notwendig, dass der Schließ-Schwellenwert explizit beispielsweise in der elektrischen Steuerung hinterlegt ist. Möglich ist auch, dass ein Schließgrad des Ventils auf monotone bzw. stetige Weise von der Beinachsenkraft abhängt, das heißt, dass das Ventil umso weiter geschlossen wird, je größer die Beinachsenkraft ist.

Weiterhin ist vorgesehen, dass in einer Variante der Erfindung das Ventil unabhängig von der Stellung der Schenkel geschaltet wird, also dass es nicht darauf ankommt, wie die Schenkel zueinander oder im Raum stehen, sondern dass lediglich darauf abgestellt wird, ob eine ausreichend große Axialkraftkomponente vorhanden ist. Ob noch Biegemomente, Torsionsmomente oder Horizontalkräfte in der Prothese oder Orthese auftreten, ist dann nicht von Bedeutung.

Eine weitere Variante sieht vor, dass das Ventil in Abhängigkeit von dem Winkel ϕ der Schenkel zueinander geschaltet wird, beispielsweise bei einem Beugewinkel zwischen 20° und 50° zueinander. Wird bei einem Einsatz in einer Beinprothese ein gewisser, festgelegter Beugewinkel erreicht, wird automatisch die Dämpfung erhöht, um zu gewährleisten, dass das Gelenk gesichert ist. Die Dämpfung kann dabei so eingestellt werden, dass ein langsames Absenken weiterhin möglich ist. Die Schaltung des Ventils aufgrund einer Verschiebung oder Verschwenkung der Schenkel zueinander erfolgt beispielsweise mechanisch, indem eine Relativbewegung der Schenkel zueinander auf das Ventil übertragen wird.

Vorteilhaft an einem erfindungsgemäßen Fluiddämpfer ist, dass er die Herstellung eines orthopädischen Hilfsmittels gestattet, das den Bedürfnissen geriatrischer Patienten entgegenkommt. Wird die Prothese belastet, steht dem Patienten eine hohe Dämpfung zur Verfügung, die ein plötzliches Einknicken vermeidet und sicheres Gehen erlaubt. Belastet verdrängt der Kolben das Fluid langsam durch den Ringspalt aus der Verdrängerkammer in das drucklose Fluidreservoir. Der Patient sinkt langsam im Kniegelenk ein und hat stets das Gefühl von der Beinprothese gestützt zu sein. Es entfällt zudem ein Entsperren des Fluiddämpfers.

Liegt hingegen an dem Kniegelenk der Beinprothese keine Belastung bzw. Beugekraft an, kann das Ventil in die Öffnungsstellung gebracht werden und das Bein schwingt widerstandsfrei durch. Es entfällt damit die Notwendigkeit, die Beinprothese außenrotiert nach vorn zu bringen oder über die Fußspitzstellung das gesunde Bein zu verlängern. Der Gang erscheint dadurch natürlicher.

Es ist ein weiterer Vorteil, dass der erfindungsgemäße orthopädietechnische Fluiddämpfer mit einfachen technischen Mitteln herstellbar ist. Bei herkömmlichen Fluiddämpfern muss eine gute Dichtwirkung zwischen Kolben und Verdrängungskammer sichergestellt werden. Eine derartige Dichtung ist aufwändig und kostenintensiv. Da erfindungsgemäß ein Ringspalt vorgesehen ist, können bei der Fertigung des Kolbens und des Zylindergehäuses deutlich geringere Fertigungstoleranzen vorgesehen werden, was die Fertigung erleichtert und verbilligt.

Aufgrund der tolerierbaren geringeren Fertigungstoleranzen ist es zudem möglich, zur Herstellung des Zylindergehäuses und/oder des Kolbens kostengünstige Werkstoffe, wie beispielsweise Kunststoff, zu verwenden, die einen kleinen Elastizitätsmodul aufweisen. Bei herkömmlichen Fluiddämpfern muss auch unter mechanischer Last die Dichtigkeit zwischen Kolben und Verdrängungskammer sichergestellt sein. Um unter mechanischer Last ein die Dichtigkeit gefährdendes Spiel zu verhindern, müssen Materialien mit einem hohen Elastizitätsmodul, wie beispielsweise Metalle, verwendet werden. Diese Notwendigkeit entfällt bei dem erfindungsgemäßen Fluiddämpfer.

Unter einem Ringspalt wird im Rahmen der vorliegenden Beschreibung insbesondere ein Zwischenraum zwischen einer Innenseite der Verdrängungskammer und einer Außenseite des Kolbens verstanden. Dieser Ringspalt kann, muss aber nicht notwendigerweise, einen kreisringförmigen Querschnitt haben. Es ist möglich, dass der Kolben bereichsweise an der Innenwand anliegt und der Querschnitt des Ringspalts daher halbmondförmig ist. Es ist eine weitere Möglichkeit, dass der Kolben in weiten Bereichen an der Innenwand anliegt und Längsnuten besitzt, durch die das Fluid von der Verdrängungskammer in das Fluidreservoir gelangen kann. Anders als bei bekannten Fluiddämpfern, bei denen Ringspalte lediglich aufgrund von Fertigungstoleranzen entstehen können, ist der Kolben beim efindungsgemäßen Fluiddämpfer so gefertigt, dass er einen Ringspalt mit vorangestellter Querschnittsfläche mit dem Zylindergehäuse bildet bzw. dass das Fluid einen voreingestellten Fluidwiderstand erfährt, wenn es durch den Ringspalt strömt. In anderen Worten bilden Kolben und Zylindergehäuse bei bekannten Fluiddämpfern eine dichtende Passung, beim erfindungsgemäßen Fluiddämpfer kann hingegen eine lockere Spielpassung gebildet sein. Daher resultieren bei bekannten Fluiddämpfern allenfalls Leckströme, nicht aber, wie beim erfindungsgemäßen Fluiddämpfer, definierte Dämpfungs-Fluidströme.

Es ist möglich, nicht aber notwendig, dass Kolben und Zylindergehäuse in allen Querschnitten senkrecht zur Kolbenlängsachse einen Ringspalt gleicher Querschnittsfläche bildet.

In einer bevorzugten Ausführungsform führt das Ventil zu einer Dämpfung der Bewegung des Kolbens in der Verdrängungskammer, wobei die Dämpfung nur in einer Bewegungsrichtung des Kolbens wirkt, beispielsweise in Einschubrichtung des Kolbens in das Zylindergehäuse. Beim Einsatz in einem orthopädischen Hilfsmittel ist es regelmäßig notwendig, aber auch hinreichend, eine Bewegung zweier Schenkel eines Gelenks des Hilfsmittels in der Einschubrichtung zu dämpfen. Wenn der orthopädietechnische Fluiddämpfer in einer Beinprothese verbaut ist, handelt es sich dabei um die Beugerichtung (Flexionsrichtung). Um das Merkmal zu verwirklichen, kann das Ventil beispielsweise ein Rückschlagventil sein, was einfach und kostengünstig ist.

Das Fluid erfährt beim Fließen entlang eines Fluidpfades aus der Verdrängungskammer durch den Ringspalt in das Fluidreservoir einen Ringspalt-Fluidwiderstand und bei einem Fluss entlang eines Fluidpfads aus der Verdrängungskammer durch die Rückstromleitung in das Fluidreservoir einen Rückstrom-Fluidwiderstand. Es ist bevorzugt vorgesehen, dass der Ringspalt eine Form und/oder eine Querschnittsfläche besitzt, die so groß bemessen ist, dass bei dem Ventil in Schließstellung der Ringspalt-Fluidwiderstand kleiner ist als der Rückstrom-Fluidwiderstand. Das heißt, dass bei geschlossenem Ventil bei einem Einschieben des Kolbens in die Verdrängungskammer mehr Fluid durch den Ringspalt in das Fluidreservoir gelangt als durch die Rückstromleitung.

Besonders bevorzugt sind das Ventil und der Ringspalt so ausgebildet, dass dann, wenn das Ventil in der Schließstellung ist und der Kolben in die Verdrängungskammer eingeschoben wird, im Wesentlichen das gesamte Fluid aus der Verdrängungskammer durch den Ringspalt in das Fluidreservoir strömt. Unter dem Merkmal, dass im Wesentlichen das gesamte Fluid auf die beschriebene Weise strömt, ist zu verstehen, dass es nicht notwendig ist, dass im strengen Sinne das gesamte Fluid durch den Ringspalt strömt. Es ist vielmehr möglich, dass ein kleiner Teilstrom weiterhin durch das Ventil strömt. Dieser Teilstrom ist beispielsweise kleiner als 15 % des Gesamtstroms.

Besonders bevorzugt ist das Fluid eine Hydraulikflüssigkeit. Diese Hydraulikflüssigkeit kann ein Öl, beispielsweise Mineralöl, aber beispielsweise auch Wasser sein. Prinzipiell möglich ist auch die Verwendung von Luft.

Günstig ist es, wenn die Viskosität der Hydraulikflüssigkeit von Magnetfeldern und/oder elektrischen Feldern unabhängig ist. Hierunter ist zu verstehen, dass sich die Viskosität insbesondere um weniger als 50% ändert, wenn ein Magnetfeld von 0,1 Tesla angelegt wird. Insbesondere ist die Hydraulikflüssigkeit magnetpartikelfrei. Derartige Hydraulikflüssigkeiten sind besonders kostengünstig.

Ein besonders einfach und kostengünstig zu fertigender Fluiddämpfer wird erhalten, wenn das Zylindergehäuse und/oder der Kolben aus Kunststoff gefertigt, insbesondere spritzgegossen sind bzw. ist. Eine weitere vorteilhafte Gestaltung umfasst ein Zylindergehäuse mit aus einer in den Kunststoff eingespritzten bzw. eingelassenen Metallhülse. Die Metallhülse bildet dabei den Zylinder. Diese Kombination verringert die Verformung des Zylinders unter Belastung. Um die alternative Verformung des Zylinders zu vermeiden, kann das Zylindergehäuse von außen mit Faserverbundwerkstoffen umfasst werden. Hierdurch wird vorteilhafterweise eine kostengünstige Massenproduktion von einfachen orthopädischen Hilfsmitteln erlaubt, die sie auch für Patienten in Ländern mit einem niedrigen Einkommen verfügbar macht.

Im Einsatz wird der Kolben des Fluiddämpfers regelmäßig in die Verdrängungskammer eingeschoben und aus dieser herausgezogen. In anderen Worten ändert sich ständig eine Einschubtiefe des Kolbens, also die Länge derjenigen Strecke, um die der Kolben in die Verdrängungskammer eingeschoben ist. Vorteilhafterweise kann die Dämpfung, die der Fluiddämpfer einem weiteren Einschieben des Kolbens in die Verdrängungskammer entgegensetzt, dadurch variiert werden, dass der Kolben eine konturierte Gestalt hat. Beispielsweise kann der Kolben konisch zulaufen, wobei sich eine Querschnittsfläche des Kolbens beispielsweise mit zunehmender Einschiebtiefe vergrößert. In diesem Fall erhöht sich der Widerstand, den der Fluiddämpfer einem weiteren Einschieben des Kolbens in die Verdrängungskammer entgegensetzt, mit der Einschubtiefe. In eine Beinprothese eingebaut bewirkt das, dass ein Beugen des Kniegelenks besonders stark gegen Ende des Hinsetzens der Patienten gedämpft wird.

Es ist zudem möglich, dass der Durchmesser des Kolbens mit zunehmender Einschubtiefe abnimmt. Möglich ist zudem, dass der Kolben konvex oder konkav ausgebildet ist. Alle genannten Formen können auch abschnittsweise vorliegen, so dass der Kolben beispielsweise einen konischen Abschnitt und einen zylinderförmigen Abschnitt besitzen kann.

Alternativ oder additiv weist zudem die Verdrängungskammer eine konturierte Gestalt auf. Hierunter ist zu verstehen, dass eine Innenwand der Verdrängungskammer wie oben geschildert konisch, konvex, konkav und/oder abschnittsweise zylinderförmig ausgebildet sein kann.

Ein besonders einfach zu fertigender Fluiddämpfer ergibt sich, wenn das Fluidreservoir ausgebildet ist, um das Fluid stets im Wesentlichen druckfrei zu speichern. Auf diese Weise wird zudem ein Verlust an Fluid weitgehend vermieden. Besonders bevorzugt ist das Fluidreservoir ein Faltenbalg. Ein derartiger Faltenbalg ist sehr leicht zu fertigen. Der Kolben weist einen Eintauchabschnitt, der in das Zylindergehäuse eintauschen kann, und einen Feinabschnitt, der nicht in das Zylindergehäuse eintauchen kann, auf. Bevorzugt ist vorgesehen, dass der Faltenbalg an dem Zylindergehäuse und dem freien Abschnitt befestigt ist. Auf diese Weise ist der Faltenbalg sicher befestigt und kann leicht für Wartungszwecke ausgetauscht werden. Grundsätzlich ist es jedoch auch möglich, das Fluid unter Druck zu speichern.

Ein durch einen Faltenbalg gebildetes Fluidreservoir ist hinsichtlich mechanischer Einwirkungen gefährdet, so dass das Fluidreservoir bevorzugt durch eine Hülse, insbesondere Kunststoffhülse umgeben ist, die vorteilhafterweise zumindest eine Druckausgleichsöffnung aufweist, damit der Faltenbalg sich ausdehnen oder eine Membran ein Aufnahmevolumen variabel ausbilden kann.

Zum Betätigen des Ventils besitzt der orthopädietechnische Fluiddämpfer bevorzugt einen außerhalb des Zylindergehäuses angeordneten Magneten, wobei das Ventil mittels des Magneten von der Öffnungsstellung in die Schließstellung bringbar ist. Die Schaltung über einen Magneten kann auch bei anderen Fluiddämpfertypen eingesetzt werden, bei denen kein Ringspalt vorhanden ist oder die doppeltwirkend oder als Rotationshydraulik aufgebaut sind. Das kann beispielsweise dadurch realisiert sein, dass der Magnet ein Elektromagnet ist, der mit einer ferromagnetischen Ventilkugel des Ventils zusammenwirkt. Das geschieht beispielsweise dadurch, dass ein Bestromen des Elektromagneten die Ventilkugel so bewegt, dass das Ventil in die Öffnungsstellung oder alternativ in die Schließstellung gebracht wird. Alternativ ist vorgesehen, dass der Magnet ein Permanentmagnet ist, der über einen außerhalb des Zylindergehäuses angeordneten Aktor oder eine mechanische Koppelung verschieblich und automatisch betätigbar gelagert ist und mit der Ventilkugel zusammenwirkt.

Ein orthopädietechnischer Fluiddämpfer, bei dem das Zylindergehäuse und/oder der Kolben aus Kunststoff gefertigt, insbesondere spritzgegossen sind bzw. ist, ist ebenfalls vorgesehen. Es kann dann vorgesehen sein, dass der Kolben über eine Dichtung, beispielsweise eine O-Ring-Dichtung, in der Verdrängungskammer gelagert ist. In diesem Fall kann neben der Rückstromleitung eine weitere Leitung vorgesehen sein, die das Fluidreservoir mit der Verdrängungskammer verbindet.

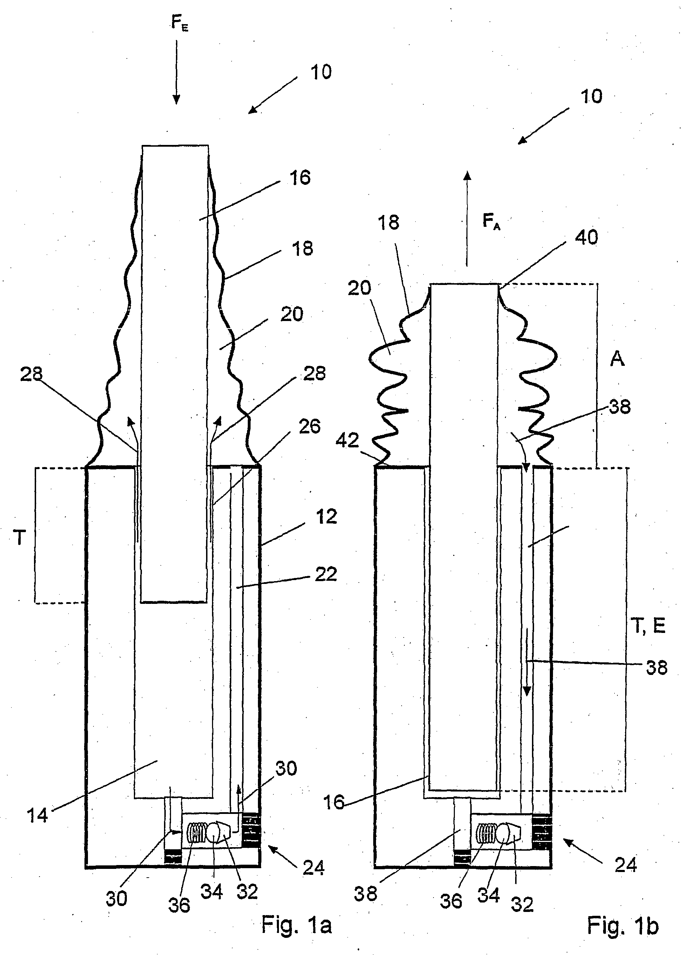

Im Folgenden werden Ausführungsformen der Erfindung anhand der beigefügten Zeichnungen näher erläutert. Gleiche Bezugszeichen bezeichnen in den Figuren gleiche oder gleichwirkende Komponenten oder Elemente. Dabei zeigt
- Figur 1a: einen erfindungsgemäßen orthopädietechnischen Fluiddämpfer in einer Querschnittsansicht, bei dem ein Kolben um eine geringe Einschubtiefe in die Verdrängungskammer eingeschoben ist,
- Figur 1 b: den Fluiddämpfer nach Figur 1a, bei dem der Kolben im Wesentlichen vollständig in die Verdrängungskammer eingeschoben ist,
- Figur 2a: eine schematische Detail-Schnittansicht des Ventils des Fluiddämpfers aus den Figuren 1a und 1b in einer Schließstellung,
- Figur 2b: das Ventil nach Figur 2a in einer Öffnungsstellung,
- Figur 3a: eine Schnittansicht eines Ventils einer weiteren Ausführungsform eines erfindungsgemäßen orthopädietechnischen Fluiddämpfers mit einer Bypassleitung, in der ein Bypass-Ventil angeordnet ist, das geschlossen ist,
- Figur 3b: das Ventil nach Figur 3 a in einer Öffnungsstellung,
- Figur 4a: eine Schnittansicht eines Ventils einer weiteren Ausführungsform eines erfindungsgemäßen orthopädietechnischen Fluiddämpfers mit einem Elektromagneten im unbestromten Zustand,
- Figur 4b: das Ventil nach Figur 4a im bestromten Zustand,
- Figur 5: ein erfindungsgemäßes orthopädisches Hilfsmittel in Form einer Beinprothese in einer gestreckten Stellung,
- Figur 6: die Beinprothese nach Figur 5 in einer gebeugten Stellung,
- Figur 7a: eine erste Ausführungsform eines Beinkraftsensors,
- Figur 7b,: eine alternative Ausführungsform eines Beinkraftsensors,
- Figur 7c,: eine weitere alternative Ausführungsform eines Beinkraftsensors,
- Figur 8a, 8b: eine Variante des Fluiddämpfers in zwei Positionen,
- Figur 9a, 9b: eine weitere Variante des Fluiddämpfers in verschiedenen Positionen,
- Figuren 10a, 10b: eine Beinprothese im unbelasteten und belasteten Zustand, sowie
- Figur 11: eine Variante der Beinprothese mit einer winkelabhängigen Hydraulikschaltung.

Figur 1a zeigt einen orthopädietechnischen Fluiddämpfer 10, der ein Zylindergehäuse 12 aus einem Kunststoff mit einer darin ausgebildeten Verdrängungskammer 14, einen in der Verdrängungskammer 14 gelagerten, aus Kunststoff bestehenden Kolben 16, ein Fluidreservoir in Form eines aus Elastomer gefertigten Faltenbalgs 18 für ein Fluid in Form einer Hydraulikflüssigkeit 20, eine in dem Zylindergehäuse 12 ausgebildete Rückstromleitung 22 und ein Ventil 24 umfasst. Der Kolben 16 ist zylinderförmig ausgebildet und unter Bildung eines Ringspalts 26 in dem Zylindergehäuse 12 gelagert. Das Ventil 24, das in Figur 1a schematisch in einer Schließstellung gezeigt ist, umfasst einen Ventilsitz 32, eine Ventilkugel 34 und eine Spiralfeder 36, die die Ventilkugel 34 auf den Ventilsitz 32 vorspannt.

Das Zylindergehäuse 12 und der Kolben 16 können alternativ auch aus Aluminium oder Edelstahl bestehen oder eine in Kunststoff eingelassene oder mit Kunststoff umspritzte Metallhülse als Laufbuchse umfassen.

Die Strecke, die der Kolben 16 in dem Zylindergehäuse 12 zurückgelegt hat, stellt eine Einschubtiefe T dar. Wenn der Kolben 16 weiter in das Zylindergehäuse 12 eingeschoben wird, so dass sich die Einschubtiefe T vergrößert, so wird in der Verdrängungskammer 14 vorhandene Hydraulikflüssigkeit 20 durch den Ringspalt 26 entlang eines Ringspalt-Fluidpfads 28 in den Faltenbalg 18 verdrängt, da ein Rückstromleitungsfluidpfad 30 durch die Rückstromleitung von dem Ventil 24 gesperrt ist.

Die Hydraulikflüssigkeit 20 erfährt entlang des Ringspalt-Fluidpfads 28 einen Ringspalt-Fluidwiderstand, der von einer Geschwindigkeit abhängig, mit der der Kolben 16 in das Zylindergehäuse 12 eingeschoben wird. Diese Geschwindigkeit hängt wiederum von einer Einschubkraft F_{E} ab, mit der der. Kolben 16 eingeschoben wird. Durch den Ringspalt-Fluidwiderstand wird die Bewegung des Kolbens 16 in das Zylindergehäuse 12 somit gedämpft.

Figur 1b zeigt einen im Wesentlichen vollständig in die Verdrängungskammer 14 eingeschobenen Kolben 16, der aufgrund einer Ausziehkraft F_{A} aus der Verdrängungskammer 14 herausgezogen wird. Aus dem Faltenbalg 18 strömt Hydraulikflüssigkeit 20 entlang eines Rückstrompfads 38 durch das Ventil 24 in die Verdrängungskammer 14. Das Ventil 24 ist als Rückschlagventil ausgebildet und ermöglicht der Hydraulikflüssigkeit 20 in dieser Richtung einen freien Durchtritt.

Figur 1b zeigt zudem einen Eintauchabschnitt E des Kolbens 16 und einen freien Abschnitt A, der nicht in das Zylindergehäuse 12 eintauchen kann. Der Faltenbalg 18 ist einerseits an einem dem Zylindergehäuse 12 abgewandten Ende 40 des Kolbens 16 und damit im freien Abschnitt A und andererseits an einer dem Ende 40 zugewandten Stirnseite 42 des Zylindergehäuses 12 befestigt. Beispielsweise ist der Faltenbalg angeklebt, angeschweißt oder er greift in jeweils nicht eingezeichneten Nuten in dem Kolben 16 bzw. dem Zylindergehäuse 12.

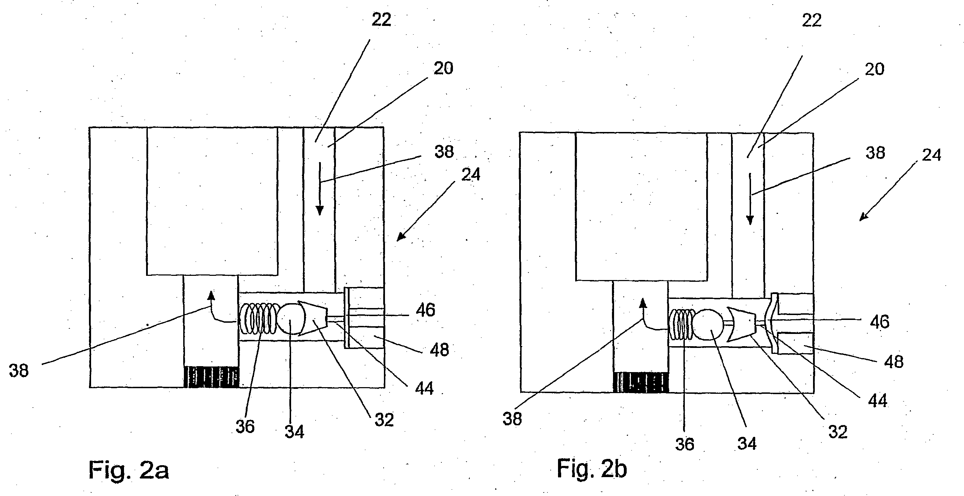

Figur 2a zeigt eine Detailansicht eines dem Kolben 16 abgewandten Endes des Zylindergehäuses 12. Es ist zu erkennen, dass die Ventilkugel 34 über einen Aktuator betätigbar ist, der einen Stift 44, eine Aktuatormembran 46 und einen Aktuatorgrundkörper 48 umfasst. Im unbetätigten Zustand, der in Figur 2a gezeigt ist, liegt die Ventilkugel 34 auf dem Ventilsitz 32 auf und verhindert einen Fluidstrom aus der Verdrängungskammer 14 in die Rückstromleitung 22 hinein. Dem hingegen kann Hydraulikflüssigkeit 20 entlang des Rückstrompfads 38 strömen, indem sie die Ventilkugel 34 entgegen der Kraft der Spiralfeder 36 vom Ventilsitz 32 abhebt.

Figur 2b zeigt den Aktor in einer Betätigungsstellung, in der der Stift 44 die Ventilkugel 34 vom Ventilsitz 32 gedrückt hat. In dieser Stellung kann Hydraulikflüssigkeit sowohl entlang des Rückstrompfads 38 als auch in Gegenrichtung das Ventil 24 passieren.

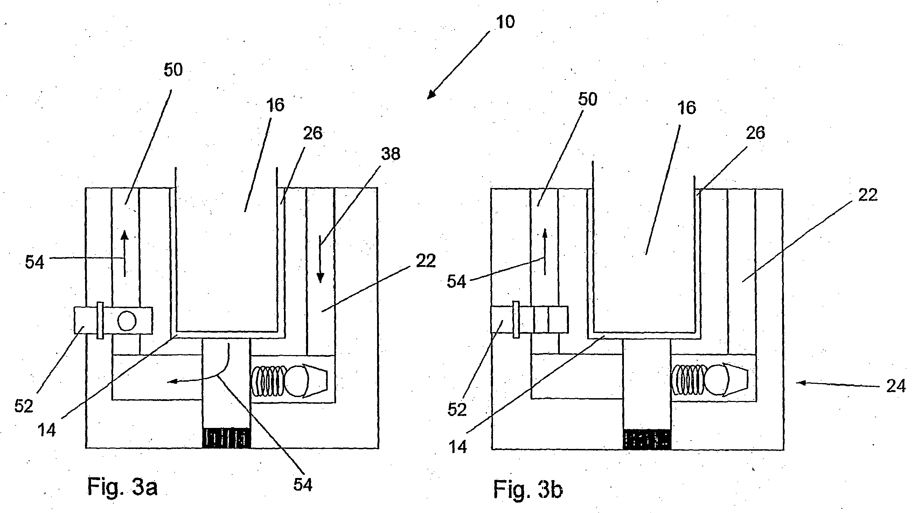

Figur 3a zeigt eine alternative Ausführungsform eines Fluiddämpfers 10, der neben der Rückstromleitung 22 eine Bypassleitung 50 besitzt, die ebenfalls die Verdrängungskammer 14 mit dem in Figur 3a nicht eingezeichneten Faltenbalg 18 verbindet. Bei dieser Ausführungsform ist kein Ringspalt 26 notwendig. In der Bypassleitung 50 ist ein Bypassventil 52 angeordnet, das die Bypassleitung 50 vollständig oder teilweise verschließen kann, so dass ein definierter Fluidwiderstand einstellbar ist. Wird der Kolben 16 aus dem Zylindergehäuse 12 herausgezogen, so kann Hydraulikflüssigkeit 20 entlang des Rückstrompfads 38 durch die Rückstromleitung 22 in die Verdrängungskammer 14 strömen. Wird der Kolben 16 in die Verdrängungskammer 14 hineingeschoben, so wird die Hydraulikflüssigkeit 20 in der in Figur 3a gezeigten Situation durch den Ringspalt 26 in den Faltenbalg (vgl. Figur 1a) verdrängt. Ein Bypassleitung-Fluidpfad 54 wird hingegen durch das geschlossene Bypassventil 52 gesperrt und die Rückstromleitung 22 ist vom Ventil 24 verschlossen.

Figur 3b zeigt die Situation, in der das Bypassventil 52 geöffnet ist, so dass beim Einschieben des Kolbens 16 in die Verdrängungskammer 14 Hydraulikflüssigkeit entlang des Bypassleitung-Fluidpfads 54 fließen kann.

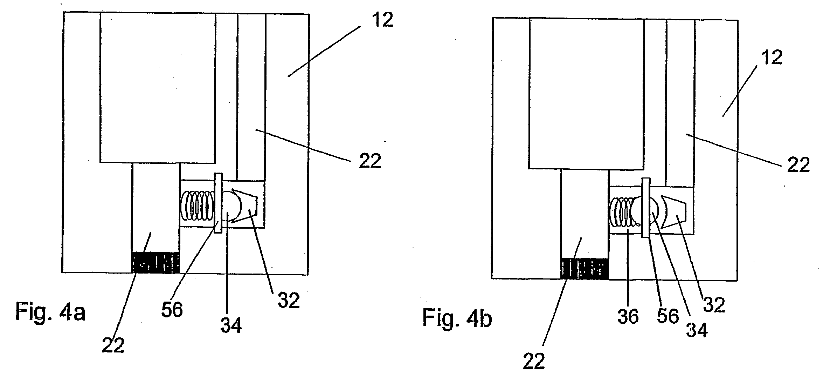

Figur 4a zeigt einen außerhalb des Zylindergehäuses 12 gelagerten Elektromagneten 56, der auf die ferromagnetische Ventilkugel 34 einwirken kann. In Figur 4a ist der Elektromagnet 56 unbestromt, so dass die Ventilkugel 34 von der Spiralfeder 36 auf den Ventilsitz 32 gedrückt wird.

Figur 4b zeigt die Situation, in der der Elektromagnet 56 bestromt ist und die Ventilkugel 34 vom Ventilsitz 32 abhebt. Der Elektromagnet 56 kann das Zylindergehäuse 12 teilweise oder vollständig ringförmig umgeben, um besonders effektiv mit der Ventilkugel 34 zusammenwirken zu können.

Alternativ ist ein Permanentmagnet vorgesehen, der mit einem Aktor verschiebbar gekoppelt ist. Eine Bewegung des Permanentmagneten mittels Aktor führt zu einer Bewegung der Ventilkugel 34.

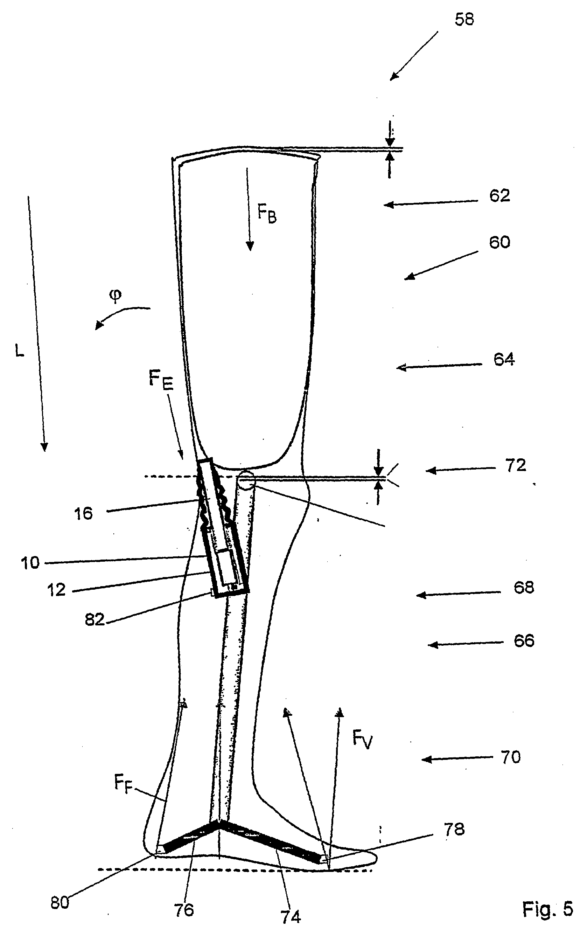

Figur 5 zeigt ein erfindungsgemäßes orthopädisches Hilfsmittel in Form einer Beinprothese 58, die einen erfindungsgemäßen Fluiddämpfer 10, einen Oberschenkel 60 mit einem proximalen Oberschenkelende 62 und einem distalen Oberschenkelende 64 sowie einen Unterschenkel 66 mit einem proximalen Unterschenkelende 68 und einem distalen Unterschenkelende 70 umfasst. Der Oberschenkel 60 und der Unterschenkel 66 sind in einem Kniegelenk 72 miteinander verbunden und verlaufen in gestreckter Stellung in einer Längsrichtung L. Der Fluiddämpfer 10 ist mit seinen Kolben 16 mit dem distalen Oberschenkelende 64 und mit seinem Zylindergehäuse 12 mit dem proximalen Unterschenkelende 68 verbunden und bewirkt eine Dämpfung einer Schwenkbewegung des Oberschenkels 60 relativ zum Unterschenkel 66 um einen Schwenkwinkel ϕ. Wenn der Oberschenkel 60 relativ zum Unterschenkel 66 schwenkt, entsteht die Einschubkraft F_{E} auf den Kolben 16 und die oben beschriebene Dämpfwirkung tritt ein.

Die Beinprothese 58 besitzt einen Vorderfuß 74 und einen Hinterfuß 76. Am Vorderfuß 74 ist ein Vorderfußkraftsensor 78 zum Messen einer Vorderfußkraft Fv angeordnet und am Hinterfuß ist ein Hinterfußkraftsensor 80 zum Messen einer Fersenkraft F_{F} montiert. Beide Sensoren stehen über eine nicht eingezeichnete elektrische Leitung in Verbindung mit einer elektrischen Steuerung 82, die Teil des Fluiddämpfers 10 ist. Die elektrische Steuerung 82 ist zudem mit einem Beinkraftsensor 84 verbunden. Der Vorderfußkraftsensor 78, der Hinterfußkraftsensor 80 und der Beinkraftsensor 84 sind ausgebildet, um eine Beinachsenkraft F_{B} zu ermitteln, die vom proximalen Oberschenkelende 62 zum distalen Unterschenkelende 70 verläuft. Die elektrische Steuerung 82 erfasst die Beinachsenkraft F_{B}**,** vergleicht diese mit einem Schließ-Schwellenwert bzw. einen Freigabe-Schwellenwert, die in einem elektrischen Speicher der elektrischen Steuerung 82 abgelegt sind, und steuert auf Basis dieses Vergleichs beispielsweise den Elektromagneten 56 (vgl. Figur 4b) an. Übersteigt beispielsweise die Beinachsenkraft F_{B} den voreingestellten Schließ-Schwellenwert, so ist dies ein Zeichen dafür, dass die Beinprothese 58 von einem Patienten belastet wird und ein hoher Beugewiderstand notwendig ist. Der Elektromagnet 56 wird dann von der elektrischen Steuerung 82 stromfrei geschaltet, so dass der Rückstromleitungs-Fluidpfad 30 (vgl. Figur 1a) für die Hydraulikflüssigkeit 20 gesperrt ist und eine hohe Einschubkraft F_{E} aufgewendet werden muss, um den Kolben 16 in das Zylindergehäuse 12 einzuschieben. Die Beinprothese 58 besitzt damit einen hohen Beugungswiderstand und gibt dem Patienten eine hohe. Sicherheit beim Stehen. Im Stand ist die Beinprothese 58 zudem geometrisch gesichert, da eine Lastlinie der Belastung vor dem Kniegelenk 72 verläuft und die Beinprothese 58 aus diesem Grund nicht beugt.

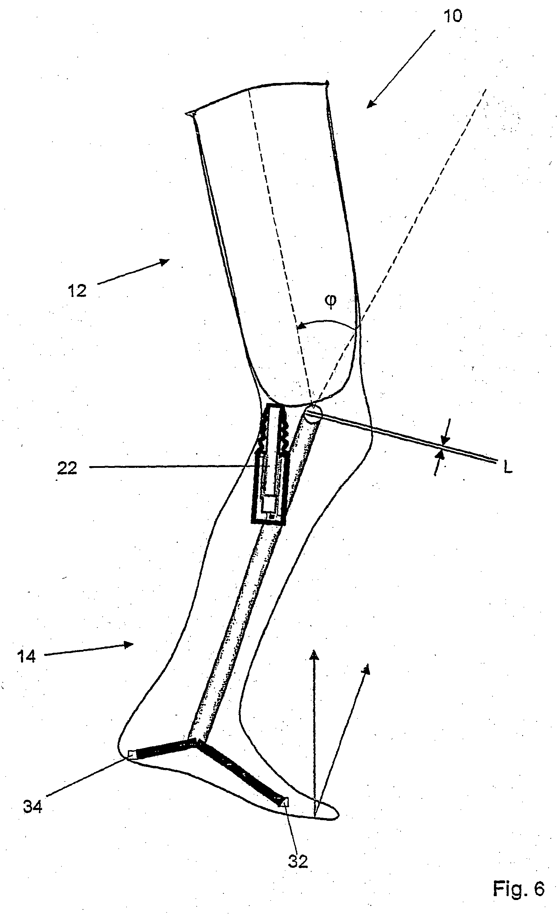

Wenn an dem Vorderfußkraftsensor 78 eine deutlich größere Kraft anliegt als am Hinterfußkraftsensor 80, so ist dies ein Zeichen dafür, dass der Patient sich setzen möchte, und die elektrische Schallsteuerung 82 schließt ebenfalls das Ventil 24. Diese Situation ist in Figur 6 gezeigt.

Wenn jedoch die Beinachsenkraft F_{B} klein ist, so ist die Beinprothese 58 unbelastet und die elektrische Steuerung 82 bestromt den Elektromagneten 56, so dass Hydraulikflüssigkeit auch durch den Rückstromleitungs-Fluidpfad 30 (vgl. Figur 1 a) strömen kann. Der Unterschenkel 66 (Figur 5) kann dann frei relativ zum Oberschenkel 60 schwingen.

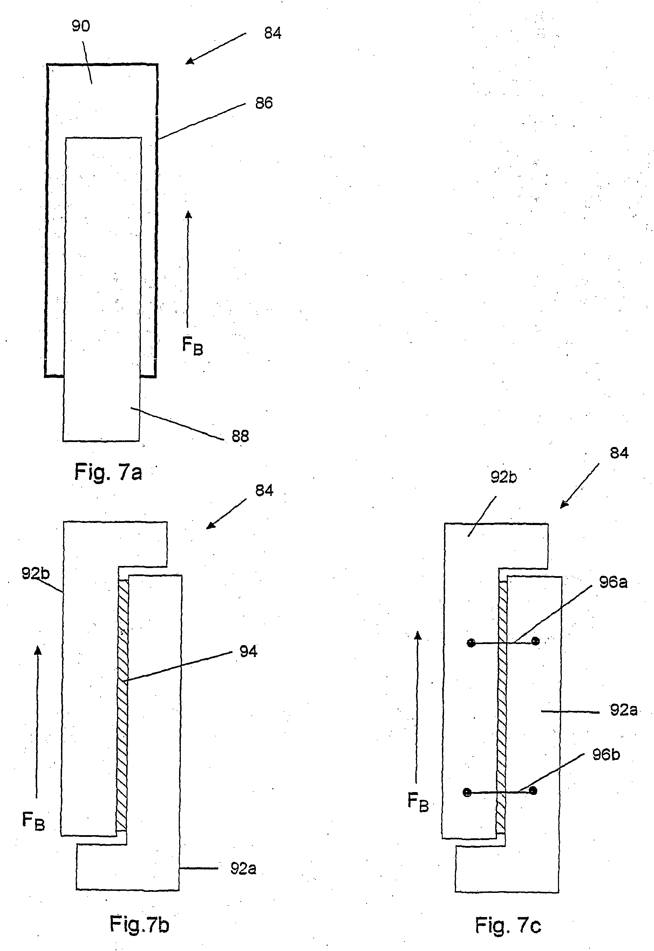

Figur 7a zeigt eine Ausführungsform des Beinkraftsensors 84 mit einer Hülse 86, in die ein Messkolben 88 gegen einen durch ein Füllmaterial 90 in einer Klebefuge ausgeübten Widerstand einschiebbar ist. Der Beinkraftsensor 84 umfasst ein Mittel zum Ermitteln der relativen Position von Messkolben 88 zur Hülse 86, die proportional zu der Beinachsenkraft F_{B} ist.

Figur 7b zeigt eine alternative Ausführungsform für den Beinkraftsensor 84, bei dem zwei L-förmige Messelemente 92a, 92b über ein elastisches Element 94 verbunden sind. Der Beinkraftsensor 84 umfasst wiederum ein nicht eingezeichnetes Mittel zum Ermitteln der relativen Position der beiden Messelemente 92a, 92b relativ zueinander, die ein Maß für die wirkende Beinachsenkraft F_{B} darstellt.

Figur 7c zeigt eine weitere alternative Ausführungsform für den Beinkraftsensor 84, der sich von dem in Figur 7b gezeigten Beinkraftsensor dahingehend unterscheidet, dass die beiden Messelemente 92a, 92b über zwei Stege 96a, 96b verbunden sind.

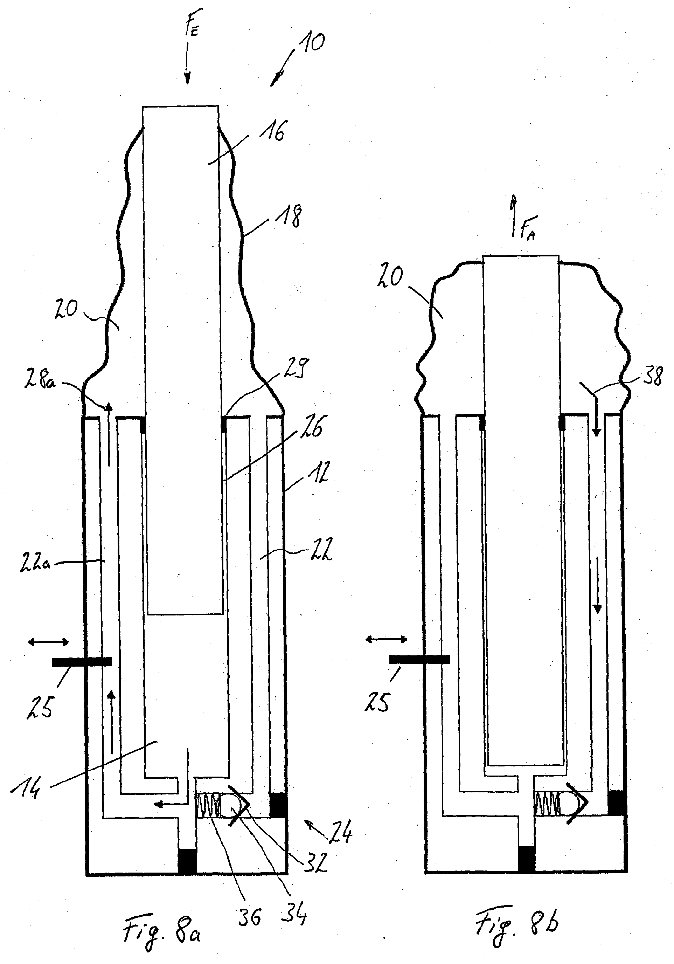

In den Figuren 8a und 8b ist ein Fluiddämpfer 10 dargestellt, der im Wesentlichen dem Fluiddämpfer der Figuren 1a und 1b entspricht. Statt des Ringspaltes 26, der geöffnet ist und ein Rückströmen der Hydraulikflüssigkeit aus der Verdrängungskammer 14 in das Fluidresevoir des Faltenbalges 18 ermöglicht, ist in der Variante gemäß der Figuren 8a und 8b der Ringspalt 26 über eine Dichtung 29 verschlossen. Statt durch den Ringspalt-Fluidpfad 28 gemäß der Ausführungsform der Figuren 1a und 1b strömt die Hydraulikflüssigkeit 20 aus der Verdrängungskammer 14 durch eine Rückströmleitung 22a, so dass sich ein Rückstromleitungs-Fluidpfad 28a ausbildet. Innerhalb der Rückstromleitung 22a ist ein Ventil 25 angeordnet, das bevorzugt als ein Magnetventil ausgebildet ist und die Rückstromleitung 22a variabel in ihrem Querschnitt verändern kann. Je weiter das Ventil 25 geöffnet ist, desto leichter kann die Hydraulikflüssigkeit 20 aus der Verdrängungskammer 14 in das Fluidreservoir des Faltenbalges 18 zurückströmen, je weiter das Ventil 25 den Strömungsquerschnitt verdrängt, desto höher wird der Widerstand gegen das Eindringens des Kolbens 16. Durch die Erhöhung der Einschubkraft F_{E} erhöht sich dann der Beugewiderstand in dem Kniegelenk der Beinprothese. Neben einer Ausgestaltung des Ventils 25 als ein Magnetventil können auch andere Ventilgestaltungen vorgesehen sein, insbesondere Stellventile, die eine schnelle und einfache Veränderung des Strömungsquerschnitts in der Rückstromleitung 22a ermöglichen.

Das Fluidreservoir ist im dargestellten Ausführungsbeispiel durch das Volumen innerhalb des Faltenbalges 18 ausgebildet, während der Fluiddämpfer 10 als ein sich linear bewegender Hydraulikdämpfer ausgebildet ist. Das Fluidreservoir kann auch in alternativen Ausgestaltungen ausgebildet sein, insbesondere kann das Fluidreservoir auch durch Druck beaufschlagt sein, so dass die Hydraulikflüssigkeit gegen einen Druck, der auch variabel sein kann, in das Reservoir hineingepresst oder aus diesem herausgepresst wird. Statt einer Ausgestaltung des Fluiddämpfers 10 als ein linearer Kolbendämpfer, kann dieser auch als eine Rotationshydraulik ausgebildet sein, bei der ein Schwenkkolben sich hin- und herschwenkend bewegt. Die beiderseits des Kolbens ausgebildeten Verdrängungskammern bilden dann das jeweilige Fluidreservoir für das durch das Gehäuse strömende Hydraulikfluid.

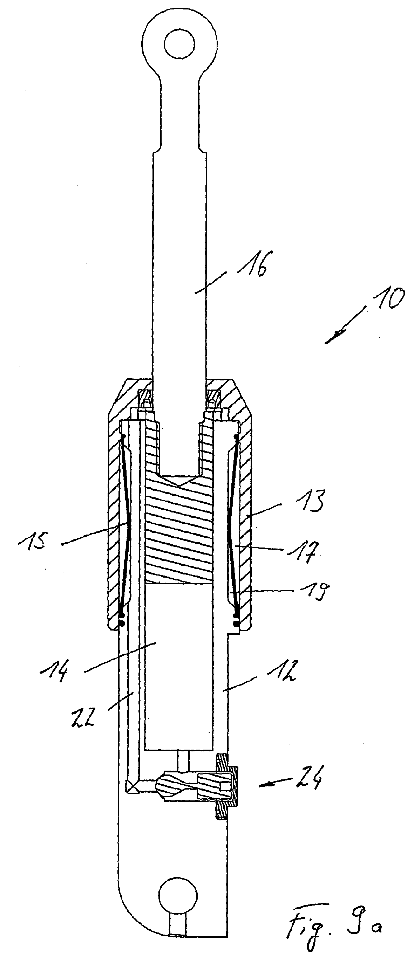

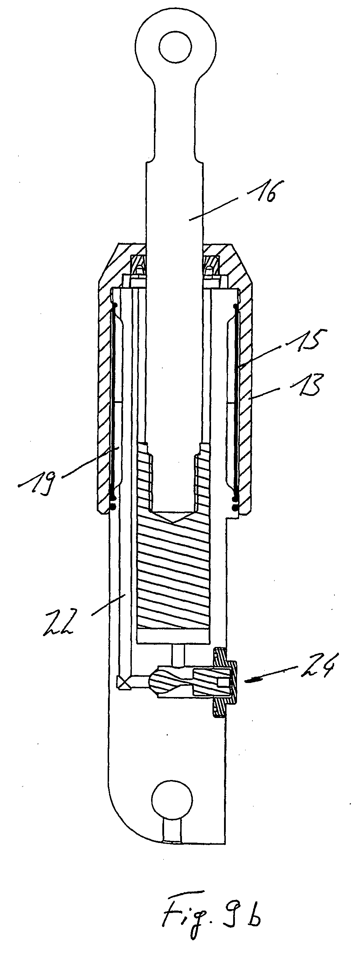

In den Figuren 9a und 9b ist eine Variante des Fluiddämpfers 10 dargestellt. Die Figur 9a zeigt den Fluiddämpfer 10 in ausgefahrener Stellung, die Figur 9b in eingefahrener Stellung. Statt eines elastischen Faltenbalges 18, wie er in den Ausführungsbeispielen gemäß den Figuren 1, 5, 6 und 8 ausgebildet ist, weist der Hydraulikdämpfer gemäß der Figuren 9a und 9b eine formstabile Schutzkappe 13 auf, die am kolbenseitigen Ende des Gehäuses 12 angeordnet ist. Statt des Faltenbalges 18 ist eine Membran 15 vorgesehen, die elastisch ausgebildet ist und innerhalb der Kappe 13 in Richtung auf das Gehäuse 12 verlagert werden kann. Die Membran 15 umschließt einen Ringspalt 19 dichtend, so dass zwischen der Membran 15 und dem Gehäuse 12 ein Fluidreservoir 19 ausgebildet ist. In der Figur 9a ist der Kolben 16 in der ausgefahrenen Stellung dargestellt, so dass die Hydraulikflüssigkeit in der Verdrängungskammer 14 gesammelt wird. Das Fluidreservoir 19 nimmt eine minimale Größe ein, was dadurch erreicht wird, dass die Membran 15 in Richtung auf das Gehäuse 12 verlagert ist. Dadurch entsteht auf der der Kappe 13 zugewandten Seite der Membran 15 ein Ausgleichsvolumen 17, das durch nicht dargestellte Löcher oder Druckausgleichsöffnungen in der Kappe 13 mit Luft aufgefüllt werden kann. Wird der Kolben eingefahren, wie in der Figur 9b dargestellt ist, wird ein gröβeres Volumen innerhalb des Fluidreservoirs 19 benötigt, so dass die Membran 15 nach außen in Richtung auf die Kappe 13 gedrückt wird. Die in dem Ausgleichsvolumen 17 vorhandene Luft wird aus der Kappe 13 herausgedrückt, wodurch sich das Volumen des Fluidreservoirs 19 vergrößern lässt. In dem dargestellten Ausführungsbeispiel ist das Fluidreservoir 19 über die Rückstromleitung 22 mit dem Hydraulikflüssigkeitskreislauf verbunden, grundsätzlich ist es auch möglich, eine entsprechende Verbindung über eine mit einem Ventil 25 versehene Rückstromleitung 22a vorzusehen.

Neben einem einseitig wirkenden Kolben 16, wie er in den dargestellten Ausführungsbeispielen gezeigt ist, ist es ebenfalls möglich, einen doppelseitig wirkenden Kolben 16 vorzusehen und in einem Hydraulikdämpfer 10 einzusetzen. Die Kappe 13 dient insbesondere zur Gewährleistung eines mechanischen Schutzes der Membran 15, die die Funktion des Faltenbalges 18 übernimmt.

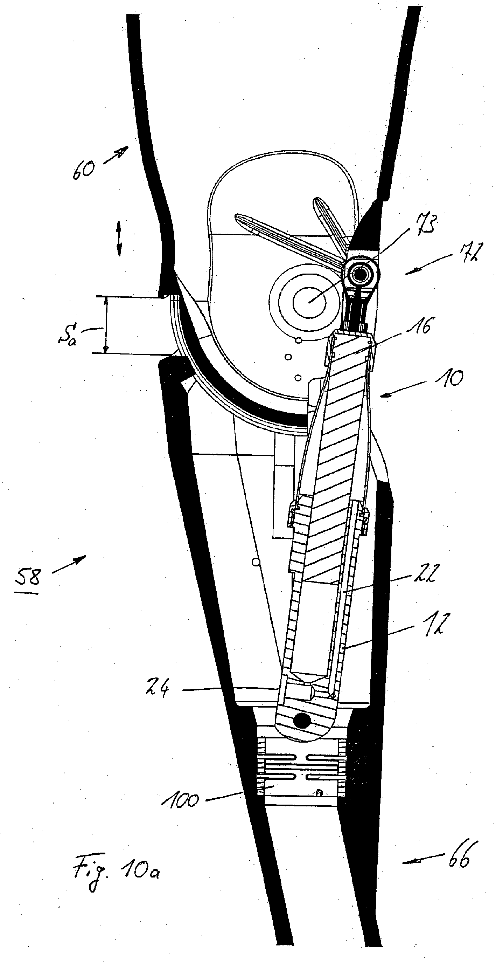

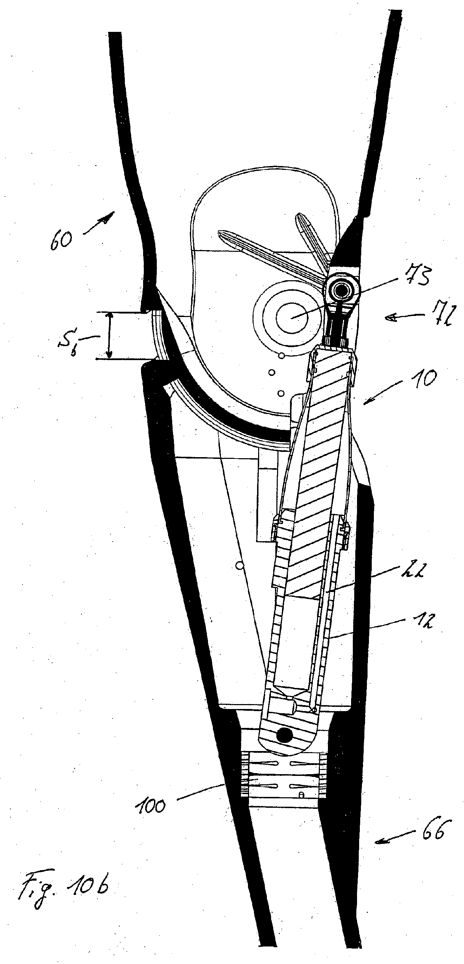

In den Figuren 10a und 10b sind verschiedene Belastungszustände einer Beinprothese dargestellt. Die Figur 10a zeigt eine Beinprothese 58 in einem unbelasteten Zustand. Ein Oberschenkelteil 60 mit einem Kniegelenk 72 und einem daran montierten Fluiddämpfer 10 ist federnd über ein am distalen Ende angeordnetes Federelement verschieblich zu dem Unterschenkel 66 gelagert. Zwischen dem Oberschenkelschaft 60 und dem Unterschenkelschaft 66 ist ein Spalt S vorgesehen, der in der Figur 10 die maximale Ausdehnung Sₐ aufweist. Wird nun eine Axialkraft auf den Unterschenkel 66 ausgeübt, federt das Federelement 100 ein, wie es in der Figur 10b dargestellt ist. Das Federelement 100 ist im dargestellten Ausführungsbeispiel als zylinderförmiges Federelement ausgebildet, dass in der Figur 10b maximal bis auf seine Blocklänge eingefedert ist. Der Spalt S ist nun minimal und in der Figur 10b mit dem Bezugzeichen S_{b} gekennzeichnet. Die Differenz zwischen Sₐ und S_{b} ist der Verschiebeweg, der im Wesentlichen dem Federweg der Feder 100 bzw. des Federelementes 100 entspricht. Durch das Einfedern des Federelementes 100 folgt eine Relativbewegung zwischen dem Fluiddämpfer 10 und dem Unterschenkel 66, die entweder für die Erzeugung eines Sensorsignals zur Schaltung eines Ventils oder für eine unmittelbare, mechanische Schaltung des Ventils ausgenutzt werden kann. Die Feder 100 bewirkt zudem eine leichte Dämpfung des Auftrittes, wobei aufgrund eines relativ kurzen Federweges kein Unsicherheitsgefühl beim Prothesenträger auftritt. Bei einer Axialbelastung, also bei einem Auftreten oder Stehen des Patienten, wird die Schaltung so bewirkt, dass ein erhöhter hydraulischer Widerstand in dem Fluiddämpfer 10 eingestellt wird, um einen Patienten ein höchst mögliches Gefühl der Sicherheit zu geben, ohne dass im entlasteten Zustand, beispielsweise im Sitzen, eine aufwändige mechanische Endriegelung des Kniegelenkes vorgenommen werden müsste. Im entlasteten Zustand gemäß der Figur 10a kanh eine Verschwenkung um die Schwenkachse 73 des Kniegelenkes 72 erfolgen, im belasteten Zustand gemäß Figur 10b ist der Widerstand wesentlich erhöht, im ldealfall hydraulisch verriegelt, so dass ohne Zerstörung mechanischer Komponenten keine Beugung möglich ist.

Die Anordnung des Federelementes 100 ist dabei sehr tief innerhalb des Unterschenkels 66 gewählt, um die Krafteinleitungsstellen möglichst weit auseinander zu legen, um die Belastungen auf die mechanischen Komponenten zu reduzieren. Die Schaltung des Fluiddämpfers 10 erfolgt unabhängig von der Orientierung der auf den Unterschenkel 66 aufgebrachten Kraft, nach Überschreiten eines Schwellenwertes eines Axialkraftanteils wird die Schaltung entweder über einen Sensor oder über eine mechanische Einrichtung wie ein Magnetventil 24, 25 ausgelöst.

Wird beispielsweise ein Fluiddämpfer 10 gemäß den Figuren 8a und 8b in der Beinprothese 58 eingebaut, bei dem das Ventil 25 als ein Magnetventil ausgebildet ist, kann eine unmittelbare Schaltung durch eine Verlagerung eines Schältmagnetes zu dem Magnetventil 25 erfolgen. Das Magnetventil 25 ist dabei in Richtung auf eine Schließstellung vorgespannt, sodass nach Wegfall einer Gegenkraft durch den Schaltmagneten das Magnetventil 25 automatisch schließt. Ist also ein Schaltmagnet in dem Unterschenkel 66 angeordnet und wird eine Einfederung des Federelementes 100 bewirkt, verlagert sich das Gehäuse 12 des Fluiddämpfers 10 relativ zu dem Unterschenkel 66 und damit das Magnetventil 25 relativ zu dem Schaltmagneten. Ist der Unterschenkel 66 ausreichend belastet, ist der Schaltmagnet soweit von dem Magnetventil 25 entfernt, dass die Schaltkraft die Vorspannung nicht übersteigt, sodass das Magnetventil 25 schließt. Wird der Unterschenkel 66 entlastet, bewegt sich durch die Rückstellkraft der Feder 100 das Gehäuse 12 in die Ausgangsstellung gemäß Figur 10 und das Magnetventil 25 wird geöffnet. Dadurch wird die Dämpfung reduziert, weil die Hydraulikflüssigkeit 20 aus der Verdrängungskammer 14 nahezu ungehindert in das Fluidreservoir im Faltenbalg 18 hineinströmen kann. In dem Ausführungsbeispiel gemäß der Figuren 10a und 10b weist der Fluiddämpfer 10 einen Faltenbalg auf, alternativ dazu kann auch ein Fluiddämpfer gemäß der Figuren 9a und 9b eingesetzt werden, mit einer Kappe 13 als mechanischer Schutz, entweder des Faltenbalges 18 oder der Membran 15. Grundsätzlich ist jedoch auch der Fluiddämpfer 10 gemäß der Figuren 1 a und 1 b möglich und vorgesehen, ebenfalls kann ein Fluiddämpfer gemäß der Figuren 1a und 1b mit einer Schutzkappe 13 gegen eine Beschädigung des Faltenbalges 18 versehen werden. Alternativ zu einem Magnetventil kann die Aktuierung eines Ventils auch durch eine Knickfeder, eine Kulissenführung oder eine Hebelmechanik erfolgen.

Alternativ zu der dargestellten Ausführungsform des Federelementes können andere teleskopierbare Einrichtungen und Federelemente vorgesehen sein, die eine Relativverlagerung des Oberschenkels 60 bzw. des Oberschenkelschaftes 60 und des Unterschenkels 66 zueinander ermöglichen. In dem Ausführungsbeispiel gemäß der Figuren 10a und 10b ist das Kniegelenkt 72 zusammen mit dem Fluiddämpfer 10 und einer nicht näher dargestellten Strebe als eine Art Rahmen ausgebildet und an dem Oberschenkel bzw. Oberschenkelschaft 60 gelagert, so dass diese Komponenten gemeinsam relativ zu dem Unterschenkel 66 verlagert werden können. Die Anordnung des Federelementes 100 im Unterschenkel 66 ist nicht zwingend, grundsätzlich kann auch eine andere Relativverlagerung zwischen Unterschenkel 66 und Dämpfer 10 realisiert werden.

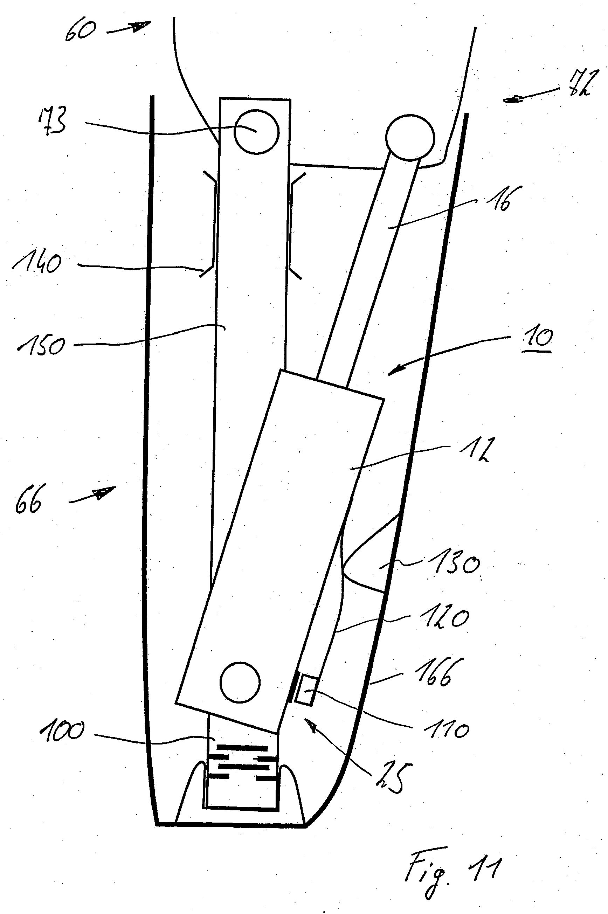

In der Figur 11 ist eine Variante der Erfindung schematisch dargestellt. Ein Oberschenkelschaft 60 ist über die Drehachse 73 des Kniegelenkes 72 schwenkbar mit einem Unterschenkel 66 verbunden. Der Unterschenkel 66 weist ein Gehäuse 166 auf, innerhalb dessen der Hydraulikdämpfer 10 sowie ein Stab 150 gelagert ist. Der Stab 150 trägt an seinem proximalen Ende die Drehachse 73 und an seinem distalen Ende das Federelement 100. Ebenfalls ist an diesem Stab 150 das distale Ende des Gehäuses 12 gelagert. Das proximale Ende des Kolbens 16 ist an dem Oberschenkelschaft 60 oder Oberschenkel 60 angeordnet. Innerhalb des Gehäuses 166 ist ein Nocken 130 angeordnet, der in Richtung auf das Dämpfergehäuse 12 hervorragt. An dem Gehäuse 12 ist ein Schaltmagnet 110 federnd über eine Federzunge 120 gelagert. Der Schaltmagnet 110 liegt dem Magnetventil 25 gegenüber.

Ebenfalls innerhalb des Gehäuses 166 ist eine Axialführung 140 für den Stab 150 vorgesehen, über die sichergestellt wird, dass der Stab 150 sich nur axial bewegt, so dass eine seitliche Verlagerung des Stabes 150 und damit des Kniegelenkes 120 und des Oberschenkels 60 oder des Oberschenkelschaftes 60 relativ zu den Unterschenkel 66 vermieden wird. Wird eine Axialkraft auf den Unterschenkel 66 ausgeübt, die in Richtung auf die Drehachse 73 eine ausreichend große Komponente aufweist, federt das Federelement 100 ein, so dass die Federlasche 120 relativ zu der Nocke 130 verlagert wird. Aufgrund der gebogenen Form der Federlasche 120 verringert sich der Druck bei einer zunehmenden Axialverlagerung in distaler Richtung, sodass der Schaltmagnet 110 durch die Rückstellkraft der Federlasche 120 zunehmend von dem Magnetventil 25 entfernt wird. Sobald der Schaltmagnet 110 ausreichend von dem Magnetventil 25 entfernt wurde, schließt das Magnetventil 25 augenblicklich und erhöht schlagartig die Dämpfung. Der gleiche Mechanismus kann auch zur winkelabhängigen Steuerung der Dämpferkraft eingesetzt werden. Wird der Oberschenkel 60 oder der Oberschenkelschaft 60 um die Schwenkachse 73 verschwenkt, führt die Lagerstelle des Kolbens 16 an dem Oberschenkelschaft 60 eine Kreisbewegung aus, die neben einer vertikalen Komponente auch eine horizontale Komponente aufweist. Über die horizontale Verlagerung des Kolbens 16 und damit einhergehend des Gehäuses 12 wird die Schaltnocke 130 von der Federlasche 120 entfernt, sodass bei einem zunehmenden Schwenkwinkel ϕ des Oberschenkels 60 relativ zu dem Unterschenkel 66 das Magnetventil 25 aktiviert wird, indem der Schaltmagnet 110 von dem Gehäuse 12 weg verlagert wird. Die dargestellte Ausführungsform hat den Vorteil, dass bei einem Versagen der Steuerung, beispielsweise wenn die Federzunge 120 bricht oder eine ungewollte axiale Verlagerung auftritt, dass die Dämpfung augenblicklich erhöht wird, sodass das Kniegelenk 72 stabil gehalten wird. Dies ist für geriatrische Patienten außerordentlich wichtig, um ein sicheres Gefühl zu erhalten.

Neben einer magnetischen Aktivierung des Ventils 25 können auch andere mechanische Kopplungen erfolgen, beispielsweise über Kurvenscheiben, Kulissenführungen oder Hebeleinrichtungen, die bei einer ausreichenden Verlagerung in axialer Richtung oder einer ausreichenden Beugung das Ventil schließen.

Das Federelement 100 ist als ein Federblock ausgebildet, der neben einer Zurverfügungstellung eines Verschiebeweges zwischen dem Oberschenkelschaft 60 und dem Unterschenkelschaft 66 auch dazu dient, Querkräfte aufzunehmen und ein eventuelles Spiel innerhalb des Gelenkes 72 oder der Prothese 58 zu vermeiden. Die Blockfeder 100 kann Querkräfte aufnehmen und stellt eine gewisse Dämpfung eines Auftrittes bereits, wobei gleichzeitig ein Anschlag und eine Begrenzung des axialen Verschiebeweges über die Feder 100 eingestellt werden kann.

Der Einsatz eines Fluiddämpfers in einem geriatrischen Kniegelenk wurde bislang als zu aufwändig angesehen, es hat sich jedoch herausgestellt, dass die Fluiddämpfer sehr gut geeignet sind, da sie keinen "Stick-Slip"-Effekt aufweisen, der bei anderen mechanischen Verriegelungen oder Bremseinrichtungen auftritt. Neben der dargestellten mechanischen Schaltung des Ventils 25 ist ebenfalls eine elektronische Erfassung eine Verlagerung oder eine Axialkraft und eine entsprechende Aktuierung des Ventils über einen Aktuator möglich. Die winkelabhängige Steuerung legt ab einem relativ geringen Beugewinkel einen relativ hohen Widerstand an das Gelenk 70 an, um relativ früh eine hohe Dämpfung und Standphasensicherheit bereit zu stellen. Die Schaltschwelle, bei der das Ventil 25 schließt und einen erhöhten Widerstand bereitstellt, kann eingestellt werden und liegt bevorzugt zwischen 20° und 50° Beugewinkel. Innerhalb dieses Winkelbereiches von 20° bis 50° wird von einem relativ niedrigen Widerstand innerhalb des Fluiddämpfers auf einen hohen Widerstand geschaltet.

| | | | |
|---|---|---|---|
| **Bezugszeichenliste** | | 50 | Bypassleitung |
| 10 | Fluiddämpfer | 52 | Bypassventil |
| 12 | Zylindergehäuse | 54 | Bypassleitung-Fluidpfad |
| 13 | Kappe | 56 | Elektromagnet |
| 14 | Verdrängungskammer | 58 | Beinprothese |
| 15 | Membran | 60 | Oberschenkel |
| 16 | Kolben | 62 | proximales Oberschenkelende |
| 17 | Freiraum | 64 | distales Oberschenkelende |
| 18 | Faltenbalg | 66 | Unterschenkel |
| 19 | Fluidreservoir | 68 | proximales Unterschenkelende |
| 20 | Hydraulikflüssigkeit | 70 | distales Unterschenkelende |
| 22 | Rückstromleitung | 72 | Kniegelenk |
| 22a | Rückstromleitung | 73 | Drehachse |
| 24 | Ventil | 74 | Vorderfuß |
| 25 | Ventil | 76 | Hinterfuß |
| 26 | Ringspalt | 78 | Vorderfußkraftsensor |
| 28 | Ringspalt-Fluidpfad | 80 | Hinterfußkraftsensor |
| 28a | Fluidpfad-Rückstrom | 82 | elektrische Steuerung |
| 29 | Dichtung | 84 | Beinachsenkraftsensor |
| 30 | Rückstromleitungs-Fluidpfad | 86 | Hülse |
| | | 88 | Messkolben |
| 32 | Ventilsitz | 90 | Füllmaterial |
| 34 | Ventilkugel | 92a, b | Messelemente |
| 36 | Spiralfeder | 94 | elastisches Element |
| 38 | Rückstrompfad | 96a, b | Stege |
| 40 | Ende | 100 | Federelement |
| 42 | Stirnseite | 110 | Magnet |
| 44 | Stift | 120 | Feder |
| 46 | Aktuatormembran | 130 | Nocken |
| 48 | Aktuatorgrundkörper | 140 | Führung |
| | | 150 | Stab |
| 166 | Gehäuse | | |
| T | | | Einschubtiefe |
| F_{E} | | | Einschubkraft |
| F_{A} | | | Ausziehkraft |
| F_{B} | | | Beinachsenkraft |
| F_{V} | | | Vorderfußkraft |
| F_{F} | | | Fersenkraft |
| E | | | Eintauchabschnitt |
| A | | | freier Abschnitt |
| L | | | Längswinkel |
| ϕ | | | Schwenkwinkel |
| S | | | Verschiebeweg |

## Patentansprüche

1. Prothese (58) oder Orthese mit einem orthopädietechnischen Fluiddämpfer (10) mit
a. einer in einem Gehäuse (12) ausgebildeten Verdrängungskammer (14),
b. einem in der Verdrängungskammer (14) gelagerten Kolben (16),
c. einem Fluidreservoir (19) für ein Fluid (20),
d. einer Rückstromleitung (22 22a), die die Verdrängungskammer (14) mit dem Fluidreservoir (19) verbindet,
e. einem Gelenk (72), das einen ersten Schenkel (60) und einen zweiten Schenkel (66) aufweist, wobei der erste Schenkel (60) mit dem Gehäuse (12) und der zweite Schenkel (66) mit dem Kolben (16) verbunden ist,
f. einem Ventil (24, 25), das eine Öffnungsstellung und eine Schließstellung, in der es die Rückstromleitung (22, 22a) zumindest teilweise verschließt, einnehmen kann, und
g. einer Vorrichtung zum Erfassen einer auf das Gelenk wirkenden Gelenkkraft (F_{B}), die ausgebildet ist, um das Ventil (24, 25) in die Schließstellung zu bringen, wenn die Gelenkkraft (F_{B}) einen voreingestellten Schwellenwert überschreitet, **dadurch gekennzeichnet, dass** der Dämpfer (10) und das Gelenk (72) gemeinsam an einem Rahmen (150, 60, 10) gelagert sind oder diesen bilden, der relativ zu einem der Schenkel (66) verlagerbar, insbesondere verschiebbar ist.

2. Prothese (58) oder Örthese nach Anspruch 1. **dadurch gekennzeichnet, dass** es eine Beinprothese (58) mit einem Kniegelenk (72) ist und die Vorrichtung zum Erfassen einer auf das Kniegelenk (72) wirkenden Gelenkkraft ein Beinachsenkraftsensor (84) zum Erfassen einer in Längsrichtung (L) der Beinprothese (58) wirkenden Beinachsenkraft ist, der ein tragendes Element der Prothese (58) ist.

3. Prothese (58) oder Orthese nach Anspruch 1 oder 2, **dadurch gekennzeichnet, dass** die Vorrichtung zur Erfassung der Gelenkkraft mechanisch aufgebaut ist und das Ventil (24, 25) bei einer Belastung in die Schließstellung verlagert.

4. Prothese (58) oder Orthese nach Anspruch **3, dadurch gekennzeichnet, dass** die Vorrichtung zur Erfassung der Gelenkkraft als eine Knickfeder, Feder (120), Nockenführung, Kulissenführung oder als ein Hebelsystem ausgebildet ist.

5. Prothese (58) oder Orthese nach Anspruch 3 oder 4, **dadurch gekennzeichnet, dass** die Vorrichtung mit einem Magnetventil (24, 25) gekoppelt ist.

6. Prothese (58) oder Orthese nach einem der voranstehenden Ansprüche, **dadurch gekennzeichnet, dass** die beiden Schenkel (60, 66) relativ zueinander begrenzt verschieblich aneinander gelagert sind.

7. Prothese (58) oder Orthese nach einem der voranstehenden Ansprüche, **dadurch gekennzeichnet, dass** an einem Schenkel (66) eine in Axialrichtung wirkende Feder (100) vorgesehen ist, die entgegen einer Verlagerung der Schenkel (60, 66) aufeinander zu wirkt.

8. Prothese (58) oder Orthese nach Anspruch 1, **dadurch gekennzeichnet, dass** zwischen dem Rahmen (150, 60, 10) und einem der Schenkel (66) eine Feder (100) angeordnet ist, die einer Verlagerung des Schenkel (66) auf den Rahmen (150, 60, 10) zu entgegen wirkt.

9. Prothese (58) oder Orthese nach einem der Ansprüche 3 bis 5, **dadurch gekennzeichnet, dass** die Vorrichtung so ausgebildet ist, dass eine von dem Schwenkwinkel ϕ abhängige Verlagerung eines Schaltelementes (110) relativ zu dem Ventil (24, 25) erfolgt.

10. **Verfahren** zum Steuern einer prothese oder Orthese nach einem der voranstehenden Ansprüche, mit den Schritten:
a. Erfassen einer auf das Gelenk (72) wirkenden Gelenkkraft (F_{B}) und
b. Ansteuern des Ventils (24, 25) so, dass es die Rückstromleitung (22, 22a) zumindest teilweise verschließt.

11. Verfahren nach Anspruch 10, **dadurch gekennzeichnet, dass** das Ventil (24, 25) unabhängig von der Stellung der Schenkel (60, 66) oder dass das Ventil (24, 25) in Abhängigkeit von dem Winkel ϕ der Schenkel (60, 66) geschaltet wird

12. Verfahren nach Anspruch 10 oder 11, **dadurch gekennzeichnet, dass** das Ventil (24, 25) mechanisch geschaltet wird, indem eine Relativbewegung der Schenkel (60, 66) zueinander auf das Ventil (24, 25) übertragen wird.

## Claims

1. Prosthesis (58) or orthesis, having an orthopaedic fluid damper (10) with
a. a displacement chamber (14) formed in a housing (12),
b. a piston (16) mounted in the displacement chamber (14),
c. a fluid reservoir (19) for a fluid (20),
d. a backflow line (22, 22a) which connects the displacement chamber (14) to the fluid reservoir (19),
e. a joint (72) which has a first limb (60) and a second limb (66), the first limb (60) being connected to the housing (12) and the second limb (66) being connected to the piston (16),
f. a valve (24, 25) which can assume an opening position and a closing position, in which it at least partially closes the backflow line (22, 22a), and
g. a device for detecting a joint force (F_{B}) acting on the joint, which is designed in order to bring the valve (24, 25) into the closing position when the joint force (F_{B}) overshoots a preset threshold value, **characterized in that** the damper (10) and the joint (72) are mounted together on a frame (150, 60, 10) or form said frame which is displaceable, in particular shiftable, in relation to one of the limbs (66).

2. Prosthesis (58) or orthesis according to Claim 1, **characterized in that** it is a leg prosthesis (58) with a knee joint (72), and the device for detecting a joint force acting on the knee joint (72) is a leg-axis force sensor (84) for detecting a leg-axis force acting in the longitudinal direction (L) of the leg prosthesis (58), which leg-axis force sensor (84) is a carrying element of the prosthesis (58).

3. Prosthesis (58) or orthesis according to Claim 1 or 2, **characterized in that** the device for detecting the joint force is set up mechanically and, in the event of load, displaces the valve (24, 25) into the closing position.

4. Prosthesis (58) or orthesis according to Claim 3, **characterized in that** the device for detecting the joint force is designed as a buckling spring, spring (120), cam guide or slotted guide or as a lever system.

5. Prosthesis (58) or orthesis according to Claim 3 or 4, **characterized in that** the device is coupled to a solenoid valve (24, 25).

6. Prosthesis (58) or orthesis according to one of the preceding claims, **characterized in that** the two limbs (60, 66) are mounted one on the other so as to be displaceable to a limited extent in relation to one another.

7. Prosthesis (58) or orthesis according to one of the preceding claims, **characterized in that** a spring (100) acting in the axial direction is provided on one limb (66) and acts counter to a displacement of the limbs (60, 66) toward one another.

8. Prosthesis (58) or orthesis according to Claim 1, **characterized in that** a spring (100) is arranged between the frame (150, 60, 10) and one of the limbs (66) and acts counter to a displacement of the limb (66) towards the frame (150, 60, 10).

9. Prosthesis (58) or orthesis according to one of Claims 3 to 5, **characterized in that** the device is designed such that a displacement, dependent on the pivot angle ϕ, of a switching element (110) in relation to the valve (24, 25) takes place.

10. Method for controlling a prosthesis or orthesis according to one of the preceding claims, with the steps:
a. detection of a joint force (F_{B}) acting on the joint (72), and
b. activation of the valve (24, 25) such that it at least partially closes the backflow line (22, 22a).

11. Method according to Claim 10, **characterized in that** the valve (24, 25) is switched independently of the position of the limbs (60, 66), or **in that** the valve (24, 25) is switched as a function of the angle ϕ of the limbs (60, 66).

12. Method according to Claim 10 or 11, **characterized in that** the valve (24, 25) is switched mechanically **in that** a relative movement of the limbs (60, 66) in relation to one another is transmitted to the valve (24, 25).

## Revendications

1. Prothèse (58) ou orthèse comportant un amortisseur à fluide orthopédique (10), comprenant
a) une chambre de refoulement (14) réalisée dans un boîtier (12),
b) un piston (16) monté dans la chambre de refoulement (14),
c) un réservoir à fluide (19) pour un fluide (20),
d) une conduite de retour (22, 22a) qui relie la chambre de refoulement (14) au réservoir à fluide (19),
e) une articulation (72) qui comprend une première branche (60) et une seconde branche (66), la première branche (60) étant reliée au boîtier (12) et la seconde branche (66) étant reliée au piston (16),
f) une vanne (24, 25) qui peut occuper une position d'ouverture et une position de fermeture dans laquelle elle referme au moins partiellement la conduite de retour (22, 22a), et
g) un dispositif de détection d'une force d'articulation (F_{B}) agissant sur l'articulation, qui est conçu pour amener la vanne (24, 25) dans la position de fermeture lorsque la force d'articulation (F_{B}) dépasse une valeur seuil préréglée, **caractérisée en ce que** l'amortisseur (10) et l'articulation (72) sont montés conjointement sur un cadre (150, 60, 10) ou forment un tel cadre, qui est mobile, en particulier par translation, par rapport à la branche (66).

2. Prothèse (58) ou orthèse selon la revendication 1, **caractérisée en ce qu'**elle est une prothèse de jambe (58) avec une articulation de genou (72), et le dispositif de détection d'une force d'articulation agissant sur l'articulation de genou (72) est un capteur (84) de force sur l'axe de jambe destiné à détecter une force d'axe de jambe agissant en direction longitudinale (L) de la prothèse de jambe (58) et qui est un élément porteur de la prothèse (58).

3. Prothèse (58) ou orthèse selon la revendication 1 ou 2, **caractérisée en ce que** le dispositif destiné à détecter la force d'articulation est de structure mécanique et déplace la vanne (24, 25) vers la position de fermeture lors de l'application d'une contrainte.

4. Prothèse (58) ou orthèse selon la revendication 3, **caractérisée en ce que** le dispositif destiné à détecter la force d'articulation est réalisé sous forme de ressort coudé, de ressort (120), de guidage à came, de guidage à coulisse ou sous forme de système à levier.

5. Prothèse (58) ou orthèse selon la revendication 3 ou 4, **caractérisée en ce que** le dispositif est couplé à une électrovanne (24, 25).

6. Prothèse (58) ou orthèse selon l'une des revendications précédentes, **caractérisée en ce que** les deux branches (60, 66) sont montées l'une sur l'autre avec faculté de translation limitée l'une par rapport à l'autre.

7. Prothèse (58) ou orthèse selon l'une des revendications précédentes, **caractérisée en ce que** sur une branche (66) est prévu un ressort (100) agissant en direction axiale qui agit à l'encontre d'un déplacement des branches (60, 66) l'une vers l'autre.

8. Prothèse (58) ou orthèse selon la revendication 1, **caractérisée en ce qu'**un ressort (100) est agencé entre le cadre (150, 60, 10) et l'une des branches (66), qui agit à l'encontre d'un déplacement de la branche (66) vers le cadre (150, 60, 10).

9. Prothèse (58) ou orthèse selon l'une des revendications 3 à 5, **caractérisée en ce que** le dispositif est réalisé de telle sorte qu'il se produit un déplacement d'un élément de commutation (110) par rapport à la vanne (24, 25), qui dépend de l'angle de basculement ϕ.

10. Procédé de commande d'une prothèse ou orthèse selon l'une des revendications précédentes, comprenant les étapes suivantes :
a) on détecte une force d'articulation (F_{B}) qui agit sur l'articulation (72), et
b) on pilote la vanne (24, 25) de manière à ce qu'elle ferme au moins partiellement la conduite de retour (22, 22a).

11. Procédé selon la revendication 10, **caractérisé en ce que** la vanne (24, 25) est commutée indépendamment de la position des branches (60, 66), ou **en ce que** la vanne (24, 25) est commutée en dépendance de l'angle (p des branches (60, 66).

12. Procédé selon la revendication 10 ou 11, **caractérisé en ce que** la vanne (24, 25) est commutée par voie mécanique en transmettant à la vanne (24, 25) un mouvement relatif des branches (60, 66) l'une vers l'autre.
